# EUROPEAN PATENT APPLICATION

(11) **EP 3 816 630 A1**
(43) Date of publication of application: **05.05.2021**
(21) Application number: 19210424.8
(22) Date of filing: 20.11.2019
(51) Int. Cl.: G01N 33/68

(54) **ANALYSIS OF PROTEIN TERMINI**

(30) Priority: 30.10.2019 EP 19206374
(71) Applicant: Christian-Albrechts-Universität zu Kiel, 24118 Kiel (DE)
(72) Inventor: Helbig, Andreas, 24105 Kiel (DE); Tholey, Andreas, 24105 Kiel (DE)
(74) Representative: Taruttis, Stefan Georg

(57) **Abstract**

The invention provides a process for analysis of terminal sections of proteins comprising the steps of
- providing one original isolated protein or a mixture of at least 2 original proteins,
- digesting the original protein or mixture of original proteins by one exopeptidase, which is a carboxypeptidase or an aminopeptidase, to generate terminal peptide sections of the isolated protein or of the mixture of proteins, and free amino acids,
- separating the terminal peptide sections from the free amino acids,
- determining the masses, and preferably additionally determining fragmentation spectra, of the terminal peptide sections by mass spectrometry, preferably by ESI-MS/MS, and
- comparing the determined masses, and preferably comparing the fragmentation spectra, to pre-determined masses of peptides to identify the amino acid sequences of the terminal peptide sections,
- from the determined masses identifying terminal peptide sections
- and assigning the terminal peptide sections having the same sequence of terminal amino acids to an individual group, and for the individual group classifying the terminal peptide sequences as belonging to one individual terminus of an original protein each.

## Description

### Analysis of protein termini

The present invention relates to a process for analysing the termini of at least one protein or of a mixture of proteins. The analytical process of the invention is suitable for use in the determination of the natural termini of at least one protein and of a mixture of proteins. The analytical process has the advantage of directly determining the natural terminal sections of proteins, without chemical derivatisations of a terminal amino acid. Further, the invention relates to an apparatus for use in the process. The process can be for analysing either the N-termini or the C-termini of at least one protein or of a mixture of proteins, or for analysing both the N-termini and the C-termini, one subsequent to the other.

### State of the art

Jiménez et al., Current Protocols in Protein Science (1999), 16.7.1-16.7.3 describe the analysis of a peptide, namely for analysis of the C-terminus of a peptide by digesting the peptide by a carboxypeptidase for different periods of time, and, using the resulting ladder of peptide fragments, by calculating the mass differences between peptide fragments determined by mass spectrometry, in order to read the original sequence from differences in mass of adjacent peaks. For analysis of the N-terminus, an aminopeptidase is used for generating peptide fragments. For the assignment of mass differences to yield the correct sequence, only an isolated peptide can be analysed.

Patterson et al., Anal. Chem. 1995, 67, 3971-3978, describe the use of matrix-assisted laser desorption ionization - time-of-flight (MALDI-TOF) mass spectrometry (MS) for analysing picomoles of an isolated protein by digestion in parallel aliquots by different concentrations of a carboxy-peptidase in wells of a target plate that is inserted into the mass spectrometer. Determination of the amino acid sequence is by calculating the mass difference between two mass peaks.

The analysis of mass differences of peptides remaining from an aminopeptidase digestion in order to determine the N-terminal amino acid sequence, respectively of peptides remaining from a carboxypeptidase digestion in order to determine the C-terminal amino acid sequence, is an indirect method that depends on the accurate measurement of mass differences in steps of single amino acid masses. It depends on the digesting reaction to also generate peptides lacking only 1, only 2, only 3 and so on, amino acids from the terminal amino acids of the original protein in order to determine the sequence of the attacked protein terminus.

Kaleja et al., J. Proteome Res 2019, 18, 2954-2964 describe protein analysis by digesting protein by the endopeptidase trypsin in H₂¹⁸O, followed by liquid chromatography coupled to mass spectrometry (LC-MS). It was found that in addition to C-termini of new trypsin-generated peptides also original C-termini of proteins carried ¹⁸O, which prevented discrimination between original ¹⁶O C-termini from tryptically generated C-termini.

Schilling, O., et al., Identification and relative quantification of native and proteolytically generated protein C-termini from complex proteomes: C-terminome analysis. Methods Mol Biol, 2011. 781: p. 59-69 describe an analytical process for isolating and identifying C-termini of proteins, which process is also termed C-TAILS.

Kleifeld et al., Nature Biotechnology 28, 281-288 (2010) describes isotopic labeling of terminal amines in protein samples for identifying N-termini and protease cleavage products, which process is also termed N-TAILS.

Colaert, N., et al., Improved visualization of protein consensus sequences by iceLogo. Nat Methods, 2009. 6(11): p. 786-7) describes a process for generating a graphical representation of similarities of biological sequences.

### Object of the invention

It is an object of the invention to provide an alternative process for analysing single proteins, preferably a mixture of proteins. This process should be able to determine the natural termini, C-termini or N-termini. Preferably, the process shall be amenable to automation, e.g. a process that allows all liquid handling in a closed system, preferably without manual handling of liquids. A further object is to provide an apparatus adapted to carry out the process.

### Description of the invention

The invention achieves the object by the features of the claims and especially provides a process for analysis of terminal sections of proteins, especially the C-terminal sections or the N-terminal sections of proteins, the process comprising or consisting of the steps of
- providing one original isolated protein or a mixture of at least 2 original proteins,
- digesting the original protein or mixture of original proteins by one exopeptidase, which is a carboxypeptidase or an aminopeptidase, to generate terminal peptide sections of the isolated protein or of the mixture of proteins, and free amino acids,
- separating the terminal peptide sections from the free amino acids, preferably by chromatography, especially by chromatography using strong cation exchange, to generate a fraction comprising or consisting of terminal peptide sections,
- preferably isolating a fraction of terminal peptide sections having a molecular size of 500 Da to 4000 Da, e.g. using size exclusion chromatography, and optionally subjecting terminal peptide sections having a higher molecular mass to an additional step of digestion by the one exopeptidase,
- preferably, from the terminal peptide sections, isolating a fraction of terminal peptide sections which are 2+ or 3+ or higher charge cations, e.g. using strong cation exchange chromatography,
- determining the masses, and preferably additionally determining fragmentation spectra, of the terminal peptide sections by mass spectrometry, preferably by ESI-MS/MS, and
- comparing the determined masses, and preferably comparing the fragmentation spectra, to pre-determined masses of peptides to identify the amino acid sequences of the terminal peptide sections,
- from the determined masses, preferably including the fragmentation spectra, identifying terminal peptide sections, which have the same terminal amino acid sequence of at minimum 5 amino acids in length
- and assigning the terminal peptide sections having the same sequence of terminal amino acids to an individual group, and for the individual group classifying the terminal peptide sequences as belonging to one individual terminus of an original protein each, wherein the step of assigning the terminal peptide sequences to an original protein is done by comparing the amino acid sequence of the individual terminal peptide sections to a database containing pre-determined amino acid sequences of proteins.

Due to the successive hydrolysis of amino acids by the exopeptidase, terminal series of peptides are generated from the isolated protein, respectively from the at least two proteins of a mixture. When a mixture of at least two proteins is analysed, the terminal peptides, which are identified to contain the same terminal amino acid sequence can be grouped to a terminal peptide series of each sample protein. Therein, the termini of the original sample proteins by multiple unique peptide sequences, which each contain the same terminal amino acid sequence of at least five amino acids using comparisons with a database containing pre-determined masses of peptides, preferably including pre-determined masses of fragmentation spectra of peptides.

Terminal peptide series preferably are determined as peptide masses, which have the same terminal portion of at least 5 terminal amino acids and assigning the terminal peptide sections having the same sequence of at least 5 terminal amino acids to an individual series, and for the individual series classifying the terminal peptide sequences as belonging to one individual terminal peptide section of one original protein each, wherein the step of assigning the individual terminal peptide sections to an original protein is done by comparing the amino acid sequence of the individual terminal peptide section to a database containing pre-determined amino acid sequences of proteins. Comparing, or matching, of experimentally found peptides to theoretical peptides in a database is e.g. done by comparing first the mass of the peptide sections as measured by mass spectrometry to masses of potential peptide matches and to then compare the detected masses of the fragmentation behaviour of the peptide section to the theoretical fragments of the potential peptide matches to find the peptide which is the most likely matching one. Therein, the theoretical fragments and their masses, which are generated by the fragmentation behaviour, are pre-determined, e.g. using an algorithm, and data on the fragmentation behaviour and the masses of the fragments, e.g. masses of the fragmentation spectra, are contained in the database and are retrieved from the database. The database containing pre-determined amino acid sequences of proteins and the database containing pre-determined masses of peptides and pre-determined fragmentation spectra can be the same or can be different databases.

In the process, an isolated protein consists e.g. of molecules of one and the same protein having the same amino acid sequence, which isolated protein is for example purified and separated from a mixture.

Herein, the proteins that are analysed by the analytical process are also termed original proteins, e.g. for differentiation from terminal peptide sections that are generated by the exopeptidase digestion from the original proteins.

The step of isolating a fraction of terminal peptide sections having a molecular size of 500 Da to 4000 Da and the step of isolating a fraction of terminal peptide sections which are 2+ or 3+ or higher charge cations can be performed by one chromatographic step, e.g. by strong cation exchange chromatography or size exclusion chromatography.

In an optional step, terminal peptide sections having a molecular size higher than 4000 Da can be subjected to additional steps of digestion by the one exopeptidase followed by again isolating a fraction of terminal peptide sections of a molecular size between 500 Da to 4000 Da. Hereby, the protein termini in the sample will be successively targeted by the exopeptidase and digested into terminal peptide sections containing the original terminus opposite to the end attacked by the exopeptidase. These terminal peptide sections are then subsequently analysed by LC-MS/MS.

The exopeptidase can be in its soluble form or can be immobilized, e.g. on a membrane or on chromatography particles as a carrier, and the mixture of original proteins and subsequently the terminal peptide sections which can e.g. be a fraction having a molecular size higher than 4000 Da are brought into contact with the same exopeptidase. Therein, the exopeptidase can be immobilized on a carrier that is arranged in a column and the original protein or the higher molecular size terminal peptide sections are caused to flow through a column containing the immobilized exopeptidase. The column can be separate columns, one for flowing through an original mixture of proteins and another one for isolating the terminal peptide sections of the higher molecular size, or it may be the same column and the mixture of proteins is pumped through this column separately, i.e. prior to, the terminal peptide sections of the higher molecular mass being pumped through.

In the step of digesting the mixture of proteins by one exopeptidase, the exopeptidase has only one catalytic activity selected from a carboxypeptidase activity and an aminopeptidase activity. Preferably, the exopeptidase cleaves individual amino acids off the protein or peptide irrespective of the kind of amino acid and irrespective of the secondary structure of the original protein. The aminopeptidase can be one kind of an aminopeptidase or a combination of at least 2 kinds of aminopeptidases, e.g. at least two different aminopeptidases. The carboxypeptidase can be one kind of a carboxypeptidase or a combination of at least 2 kinds of carboxypeptidases, e.g. at least two different carboxypeptidases. A preferred carboxypeptidase is carboxypeptidase Y (CPY). A preferred aminopeptidase is aminopeptidase 1 (API), e.g. originating from S. griseus.

As the process contains the step of digesting the mixture of proteins by one exopeptidase, which is a carboxypeptidase or an aminopeptidase, this step, in the embodiment of the exopeptidase being a carboxypeptidase, generates N-terminal peptide sections of the mixture of proteins. In the embodiment of the exopeptidase being an aminopeptidase the process generates C-terminal peptide sections of the isolated protein or of proteins of the mixture of proteins. In the embodiment of the exopeptidase being a carboxypeptidase, the process identifies the amino acid sequences of N-terminal peptide sections. In the embodiment of the exopeptidase being an aminopeptidase, the process identifies the amino acid sequences of C-terminal peptide sections. The amino acid sequences of the terminal peptide sections are identified by comparison of the masses and preferably by the fragment ions that were determined for the terminal peptide sections to masses of known protein sequences, which are contained in a database.

Modifications of the terminal amino acids of the original proteins contained within the sample protein or within the sample mixture of proteins by the process are determined by the generation of terminal peptide sections using the exopeptidase digestions. The process applied to such modified termini generates terminal peptide sections with shifted masses compared to the unmodified proteins. When including the possibility for the presence of such terminal modifications (e.g. N-acylation) in the subsequent database searches this will allow their identification due to the mass shift and matching fragment ions in the MS2 spectra. The process has the advantage that the terminal peptide sections contain the original terminal modifications and that the determination of the masses, preferably also the determination of fragmentation spectra, of the terminal peptide sections allows the identification of terminal modifications by comparison to pre-determined masses of peptides and fragmentation spectra, respectively.

Preferably, in the embodiment of the exopeptidase being a carboxypeptidase, free amine groups of the mixture of proteins can be alkylated, e.g. methylated, e.g. dimethylated, prior to digesting the mixture of proteins by the carboxypeptidase for generating methylated, e.g. dimethylated, N-terminal peptide sections. Such chemical modifications can be employed to label the original N-terminus of the sample proteins to verify the N-terminal sequences obtained by the described process. Accordingly, optionally, the process includes chemically modifying, e.g. alkylating free amino groups of an aliquot of the isolated protein or of the mixture of at least two proteins, and performing the same process steps on the modified, e.g. alkylated, protein and mixture of proteins, respectively. Dimethylation can e.g. be done by incubating the mixture of proteins with formaldehyde and cyanoborohydride. The alkylation, e.g. methylation or dimethylation, results in methylated, e.g. dimethylated, amines of originally free amine groups, e.g. of N-termini and lysine side chains. Chemical modification of free amino groups of original proteins can alternatively be carried out by isobaric labelling reagents that allow the comparison of multiple (e.g. up to 11) biological samples using reporter ions generated during peptide fragmentation and which reagents are visible in the low mass region of the generated MS/MS spectra. These reporters allow quantification of relative peptide abundance between biological samples. The most commonly used commercial isobaric mass tags are the TMT (tandem mass tags) system from Thermo Fisher Scientific and the iTRAQ system from AB SCIEX UK Ltd, Warrington, Cheshire, GB.

Preferably, in the embodiment of the exopeptidase being aminopeptidase, the step of digesting the mixture of proteins can be performed in H₂¹⁸O, resulting in the C-termini generated by the process bearing an ¹⁸O, whereas the original C-termini of the proteins and the C-terminal peptide sections generated by the aminopeptidase digestion are solely identified as containing the original ¹⁶O.

Optionally, the original isolated protein, respectively the mixture of original proteins can be reduced and further optionally alkylated, generating a mixture of proteins that is free of disulphide bonds. Reducing the isolated protein, respectively the mixture of original proteins can be achieved by reacting these with a reducing agent.

The analytical process of the invention accordingly has the advantage of directly measuring the original terminal amino acids of terminal protein sections of original proteins. It was found that the process is suitable for analysing an isolated protein or a mixture of at least 2 or more original proteins. The mixture of original proteins can be an extract, containing all of the proteins or a fraction of a biological sample. The mixture of proteins can e.g. comprise or consist of at least two variants of essentially, e.g. at least 95 or 99%, the same amino acid sequence of one protein originating from one coding sequence, e.g. processing variants like splicing variants, N-terminal or C-terminal posttranslational modifications, e.g. acetylation, or any modification that changes, or shifts, the molecular mass. The process is usable for analysis of a mixture of at least two original proteins, because the terminal peptide sections, are assigned to an individual series of terminal peptide sections belonging to one original protein. It was found that the process generates a sufficient number, e.g. at least 2, at least 3, at least 4 or more terminal peptides from each individual original protein of the mixture. Furthermore, the step of comparing the determined masses to pre-determined masses of known peptides, e.g. to masses of peptides contained in a database, is sufficient to identify the amino acid sequences of the terminal peptide sections, and is also sufficient to assign the individual terminal peptide sections to a series each determining one original protein.

The biological sample can e.g. be a blood sample, e.g. whole blood, serum, or a cellular fraction from blood, a cell lysate, a sample of a biotechnological process, e.g. a monoclonal or polyclonal antibody sample originating from a cell culture, or a sample of a pharmaceutical compound or composition or of a food.

Generally, it is preferred that for comparing amino acid sequences, the pre-determined amino acid sequences are stored and can be retrieved from a database. The database may comprise or consist of amino acid sequences that were pre-determined by computing amino acid sequences that are encoded in pre-determined nucleic acid sequences, which preferably contain the genome of an organism, preferably of the organism that is the origin of biological sample, e.g. of the isolated protein or of the mixture of original proteins, analysed by the process of the invention.

In the analytical process, only exopeptidases are used in order to generate terminal sections of original proteins. For analysing the C-termini of original proteins, an aminopeptidase is used in order to generate C-terminal sections of the original proteins, wherein the C-terminal sections due to the absence of a chemical derivatisation of the C-terminus still contain the original C-terminal amino acid sequence including its original natural modifications.

Optionally, in the embodiment of the exopeptidase being an aminopeptidase, the mixture of proteins can be subjected to removal of an N-terminal acetylated amino acid, e.g. by incubation of the mixture of proteins with Pfu N-acetyl deblocking aminopeptidase, to increase the efficacy of the N-terminal digestion.

For analysing the N-termini of original proteins, a carboxypeptidase is used in order to generate N-terminal sections of the original proteins, wherein the N-terminal peptide sections contain the N-terminal amino acid sequence of the original proteins.

It was found that the aminopeptidase does not introduce the ¹⁸O isotope into the original C-terminus of peptides when the digestion reaction is carried out in H₂¹⁸O. Therefore, optionally, the digestion reaction with aminopeptidase is carried out in H₂¹⁸O, and determining original C-termini by containing the ¹⁶O isotope, and, optionally, determining peptides containing C-termini with the ¹⁸O isotope, which are excluded from a comparison of measured masses with a database of peptide masses, which preferably are in-silico generated, i.e. computer generated, peptide masses. In-silico generated peptide masses are mass data of peptides, including mass data of fragmentation spectra that are determined by computer-generated analysis of genomic coding DNA sequences translated into encoded amino acid sequences, optionally including computer-generated analysis of mass data, including mass data of fragmentation spectra, of amino acid sequences contained in a database. It is an advantage of the analytical process that a mixture of two or more proteins can be analysed for identifying the original protein termini of the mixture, because it is not necessary to separate the exopeptidase from the mixture of proteins. In detail, the presence of the exopeptidase in the mixture of proteins to be analysed does not impair the identification of the original proteins, e.g. because also peptides originating from impurities or impurities contained in the exopeptidase are identified by the process independently from the identification of original proteins of the mixture.

Generally, the analytical method can be performed without labelling proteins.

Preferably, in the step of comparing masses determined for terminal peptide sections, preferably also masses of fragmentation spectra, with pre-determined masses contained in a database, settings of the databank that reflect protease specificities, e.g. amino acid sections corresponding to cleavage sites, can be adjusted. For example, in the case of using unspecific enzymes such as unspecific aminopeptidases or carboxypeptidases in the process according to the invention, the settings of such a search algorithm are set to "no enzyme".

The apparatus for use in performing the analytical process comprises a mass spectrometer and at least one column containing one immobilized exopeptidase, preferably at least one column containing an immobilized exopeptidase, which is an aminopeptidase, and at least one column containing an immobilized exopeptidase, which is a carboxypeptidase. These columns are referred to as exopeptidase columns. At least one exopeptidase column is connected to a pump and a first valve that is set up for the injection of a mixture of at least 1 or more sample proteins into the at least one exopeptidase column. A buffer container connected to the pump is set up for providing buffer for pumping the mixture through the, at least one, exopeptidase column. The outlet of the exopeptidase column is connected to the mass spectrometer, which preferably is an electrospray-ionization mass spectrometer (ESI-MS/MS), with an intermediate reversed phase liquid chromatographic system. The mass spectrometer is coupled to a computer for transfer of data of determined masses, the computer having access to a database containing pre-determined mass data of proteins. The database can e.g. be UniProt or a similar source of protein sequences.

Preferably, between the outlet of the exopeptidase column and the inlet of the mass spectrometer, there is a connected size exclusion chromatography column or a strong cation exchange chromatography column. Preferably, the strong cation exchange chromatography column has size exclusion chromatography properties, or a size exclusion chromatography column is arranged between the outlet of the exopeptidase column and the mass spectrometer, upstream or downstream of the strong cation exchange chromatography column.

The strong cation exchange chromatography column is set up for separating 1+, 2+, and higher charged peptide cations using the buffer of a gradient buffer system, preferably a salt gradient buffer system, which is arranged to provide an increasing salt gradient buffer flow.

Preferably, a second valve is arranged downstream of the size exclusion chromatography column, preferably controlled by a detector arranged in the connecting tube. This second valve is set up to guide a fraction exiting the size exclusion chromatography column or exiting the strong cation exchange chromatography column to the mass spectrometer, or into an exopeptidase column. Preferably, the second valve is controlled, e.g. by the signal of the detector, to guide the first eluted peptide fraction to an exopeptidase column and to guide a second fraction towards the mass spectrometer. The second fraction is e.g. a size fraction of 500 to 4000 Da, and the first fraction is e.g. a fraction having a higher molecular size. The detector can be set to discriminate the first fraction from a second fraction by pre-determined retention times of the fractions, e.g. the second fraction having a longer retention time than the retention time of the first fraction, which is a flow through fraction of the size exclusion chromatography column.

As the outlet of the exopeptidase column via the first valve is coupled to the inlet of the strong cation exchange chromatography column or to the inlet of the size exclusion chromatography column, the apparatus is set up to guide the first fraction through an exopeptidase column, and again through the strong cation exchange chromatography column or through the size exclusion chromatography column. Accordingly, the apparatus is set up to generate terminal peptide sections of original, large molecular mass proteins while producing terminal peptide sections of different molecular sizes that can be differentially retained on the size exclusion (SEC) or strong cation exchange (SCX) column compared to the original proteins. By employing automated cycles of exopeptidase digestion and SCX or SEC separations, terminal peptide sections that form a series of sample protein sections over a large molecular weight range of the original proteins can be generated and analysed.

The apparatus preferably contains one exopeptidase column connected between the first valve and the second valve and, connected to different ports of the first valve and of the second valve, one strong cation exchange chromatography column and/or one size exclusion chromatography column in order to guide a low molecular weight peptide fraction exiting from the strong cation exchange chromatography column or from the size exclusion chromatography column to the mass spectrometer and to guide a second high molecular weight peptide fraction exiting from the strong cation exchange chromatography column or from the size exclusion chromatography column to the exopeptidase column.

Further preferred, an exopeptidase column is connected between ports of the first valve and of the second valve, and a size exclusion chromatography column is connected between different ports of the first valve and of the second valve, such that the second valve can guide a fraction exiting the size exclusion chromatography column to the mass spectrometer or back to the exopeptidase column. The outlet of the exopeptidase column is connected to the inlet of the size exclusion chromatography column, e.g. via the first valve. A strong cation exchange chromatography column or a reversed phase column, which is preferably connected to a port of the second valve, is preferably arranged between the outlet of the size exclusion chromatography column and the mass spectrometer. Generally, the strong cation exchange chromatography column is coupled to a second pump which is set up to pump an eluent having a gradient through the chromatography column in order to achieve peptide separation prior to mass spectrometric analysis. Using strong cation exchange, peptides can be fractionated according to their in-solution cationic charge of +1, +2 or a higher using a salt (e.g. KCL) gradient, while a process using a reversed-phase column would employ organic solvent to separate peptides based on their hydrophobicity.

Preferably, a desalting column is connected between the outlet of the size exclusion chromatography column and the inlet of the mass spectrometer. This desalting column serves to bind the terminal peptide sections eluting from the size exclusion column on a reversed phase column, e.g. a C18 column, and to desalt them prior to fractionation and mass spectrometric analysis.

In comparison to prior analytical methods, e.g. charge-reversal derivatization according to Kaleja et al., J. Proteome Res 2019, or Schilling, O., et al. 2011, the analytical method of the invention identifies a higher number of individual proteins from a complex mixture, e.g. from total proteins of a yeast lysate, which individual proteins are identified either by their N-termini or by their C-termini, or by both. Through the unspecific and sequential digestion using one exopeptidase protein termini are generated, which can be identified by a series of multiple unique peptide sequences, increasing confidence of the identified termini. Limitations regarding the generation of terminal peptide fragments by commonly used endoproteases such as trypsin and the subsequent enrichment of such terminal peptides using chemical enrichment procedures can also be avoided by the process according to the invention.

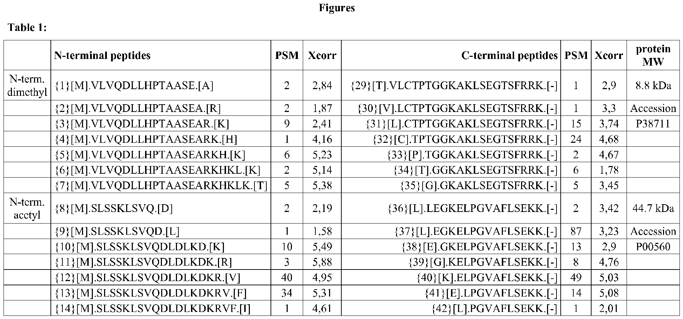

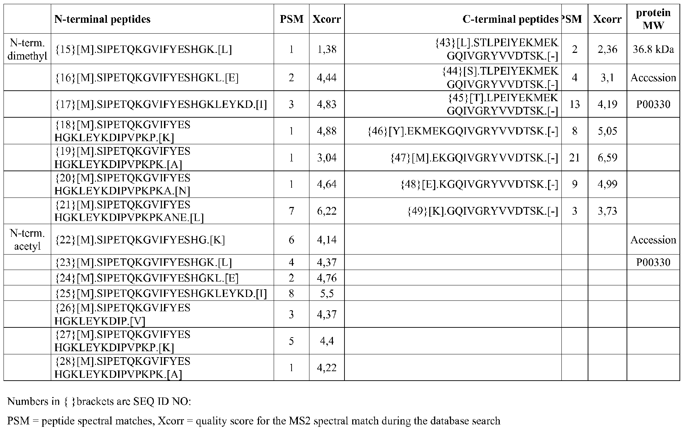

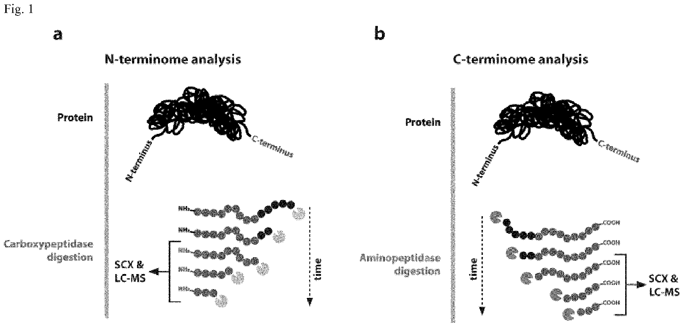

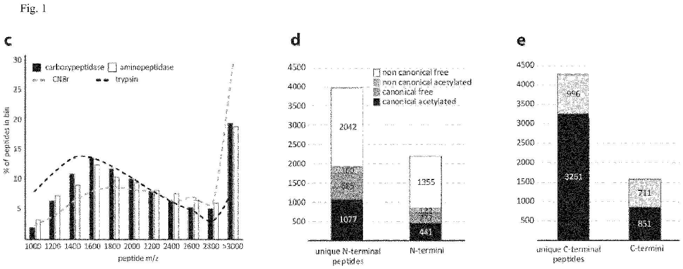

The invention is now described in greater detail by way of examples with reference to the figures, which schematically show in
- Fig. 1 a) and b) a representation of an embodiment of the process using exopeptidases in identifying protein termini
- Fig. 1 c) a histogram of peptide section size distributions,
- Fig. 1 d) and e) charts summarizing numbers of identified peptides,
- Fig. 2 a), b) and c) exemplary mass spectrometric results for individual peptides, showing the validity of aminopeptidase digestion combined with ¹⁸O incorporation to label C-terminal peptides,
- Fig. 3 in a) to b) shows cleavage preferences of the utilised aminopeptidase and carboxypeptidase. Fig. 3 c) to d) show results of the coverage of yeast lysate proteins by multiple peptides,
- Fig. 4 shows the performance of comparative methods such as N-TAILS and C-TAILS to analyse terminal peptides from a yeast lysate and the ability of the invention to increase confidence of identified termini by providing multiple unique peptide sequences per terminus,
- Fig. 5 in a) and c) shows detection of protein digestion by UV, and in b) and d) shows histograms of the number of peptide sections identified in digestion fractions separated according to charge,
- Fig. 6 schematically shows a device for performing the analytical process to determine protein termini in an automated fashion,
- Fig. 7 a further device for performing the analytical process, and
- Fig. 8 a further device for performing the analytical process.

Fig. 1 schematically shows steps of the analytical process using Fig. 1 a) carboxypeptidase for N-termini (N-terminome analysis) or Fig. 1 b) aminopeptidase for C-termini (C-terminome analysis) as the exopeptidase on the example of cell lysate as the mixture of at least 2 proteins. Following cell lysis for producing free proteins, optionally N-terminal labelling can be made, e.g. reductive dimethylation, prior to carboxypeptidase digestion. With increase in digestion time or increase in exopeptidase concentration, the molecular size of the original proteins from which terminal peptide sections are being generated, decreases (as indicated by the arrow "time"). With increase in exopeptidase digestion time or increase in exopeptidase concentration, the terminal peptide sections that are generated become shorter. The terminal peptide sections, N-terminal peptide sections in the case of carboxypeptidase digestion or C-terminal peptide sections in the case of aminopeptidase digestion, can be combined. As preferred, the terminal peptides are then separated by strong cation exchange chromatography (SCX), which in addition to separation according to cation charge also results in size separation. The subsequent liquid chromatography and ESI-MS/MS (LC-MS) determines the masses of the terminal peptides, and comparison of these determined masses with a database identifies the amino acid sequences of the terminal peptides. It was found that terminal peptides, which are identified to contain the same amino acid partial sequence at their termini can be grouped to a terminal peptide series, which can be assigned to one original protein that contains this terminal peptide amino acid sequence. For the exemplary mixture of proteins analysed, exemplary terminal peptide sequences of an individual group are shown as SEQ ID NO: 50 to SEQ ID NO: 60 for the analysis depicted in Fig. 1 a), and as SEQ ID NO: 61 to SEQ ID NO: 71 for the analysis depicted in Fig. 1 b).

### Example: Analysis of total proteins of Saccharomyces cerevisiae

As an exemplary mixture of proteins, a lysate containing the total proteins was prepared from Saccharomyces cerevisiae, strain BY4742 (Euroscraf) cultivated in synthetic yeast nitrogen base medium to OD₆₀₀ = 3, washed in phosphate buffered saline, 300 mg per replicate lysed in 6 M guanidine hydrochloride, 100 mM HEPES, pH 7.5, 2 x complete protease inhibitor and glass beads agitated at 30 Hz, followed by incubation on ice and sonication, 2 cycles of 100 % (Bandelin Sonoplus) power for 10 s each, with removal of cell debris by centrifugation at 21 000 x g for 10 min at 4 °C. For reduction of disulphide bonds, dithiothreitol was added to 10 mM, followed by alkylation by adding 55 mM iodoacetamide for 1 h at RT. The lysate was digested with exopeptidase, namely in one batch with the aminopeptidase 1 (API) from S. griseus in buffer set up in H₂¹⁸O, and in one separate batch with the carboxypeptidase Y from S. cerevisiae (CPY). Prior to the CPY digestion, protein termini were blocked by reductive dimethylation using 30 mM formaldehyde and 30 mM cyanoborohydride in overnight incubation and further addition of 20 mM formaldehyde and cyanoborohydride for another 2 h in order to label these for discrimination from termini generated by possible contaminating protease activities. The dimethylation reaction was quenched by adding 200 mM ammonium bicarbonate and incubation for 1 h. The dimethylated proteins and non-dimethylated sample aliquots were precipitated by 10 x vol. ethanol of -20°C and incubation at -80°C for 4 h, followed by centrifugation at 21 000 x g for 30 min, followed by 3 washing steps in ice-cold ethanol and subsequent drying to generate the total proteins.

Digestion reactions on the total proteins were carried out for 7 days while aliquots were removed every 12 h.

For API digestion, 1 mg of the total proteins was dissolved in 100 µL 8M guanidine HCl, 100 mM HEPES, pH 7.5. Guanidine HCl was diluted to 0.8 M using 100 mM HEPES pH 7.5. After the addition of 0.5 mM CoCl₂, 30 µg of Pfu N-acetyl Deblocking Aminopeptidase from *P. furiosus* the sample was incubated at 85 °C for 2 h. 70 µL of the reaction were removed while the was rest incubated with 30 µg API and 10 mM CaCl₂. Incubation was performed for 7 days at 37 °C with aliquots (70 µL) sampled every 12 h, combined with the previous aliquots and frozen. After every two days, another 30 µg of enzyme was added to the reaction. The combined aliquots were acidified to pH 2 by the addition of 5% formic acid. Peptides were purified using the SepPak (Waters, UK) column system (50 mg of Cis material). After binding on the column, peptides were washed using 5 % formic acid and eluted with 2 x 100 µL 80 % and 100 % acetonitrile. Peptides were lyophilized and reconstituted in 5 mM KH₂PO₄, 30 % acetonitrile (ACN), pH 2.7.

For CPY digestion, 1 mg of dimethylated and precipitated proteins were dissolved in 100 µL 8M urea in 50 mM ammonium citrate pH 6 containing 5 mM EDTA. Urea was diluted to 0.8 M using 50 mM ammonium citrate pH 6, 5 mM EDTA. 25 µg of CPY was added and the sample was digested for 7 days at 25 °C. Aliquots (70 µL) were sampled every 12 h, combined with the previous aliquots and frozen. After every two days, another 25 µg of enzyme was added to the reaction. The combined aliquots were acidified to pH 2 by the addition of 5% formic acid. Peptides were purified using the SepPak (Waters, UK) column system (50 mg of Cis material). After binding on the column, peptides were washed using 5 % formic acid and eluted two times with 100 µL 80 % and 100 % acetonitrile. Peptides were lyophilized and reconstituted in 5 mM KH₂PO₄, 30 % ACN, pH 2.7.

The respective digested proteins were separated by strong cation exchange chromatography on a Dionex Ultimate 3000 (Thermo Scientific) HPLC device using a 200 x 2.1 mm, 5 µm, 300 Å polysulfoethyl A column (PolyLC Inc, USA). Samples were loaded in eluent A (5 mM KH₂PO₄, 30 % ACN, pH 2.7 HCl) and peptides were subsequently eluted by a KCl gradient (eluent B: as A and containing 350 mM KCl) and NaCl (eluent C: as A and containing 800 mM NaCl) at a flow rate of 250 µL/min. Gradient: isocratic 0 % B from 0 to 10 min, followed by a linear ramp to 20 % B up to 40 min, 30 % B up to 50 min, 50 % B up to 55 min, 100 % B at 60 min. At 69 minutes the system was switched to 100 % C for 5 min. Peptide in-solution charge states were collected as following: +1 (0 to 23 min), +2 (23 to 38 min), +3 (38 to 53 min) and cation > +3 (53 to 78 min). Fractions were dried, re-dissolved in 5 % formic acid, desalted using Sep-Pak C18 cartridges and lyophilized to dryness.

5 µL sample volume was injected and loaded onto a trap column (Acclaim Pepmap 100 C18, 5 mm x 300 µm, 3 µm, 100 Å, Dionex).

Subsequently, size exclusion chromatography was performed on an analytical column (Acclaim Pepmap 100 C18, 50 cm x 75 µm, 2 µm, 100 Å, Dionex) at a flow rate of 300 nL/min. Eluent A (0.05 % formic acid) and eluent B (80 % acetonitrile, 0.05 % formic acid) were utilized during the following elution: linear gradient from 5 % to 20 % B in 120 min, 20-50 % B in 60 min, 50-90 % B in 8 min, 90 % B for 9 min, 90-4 % B in 1 min and equilibration with 4 % B for 15 min.

LC-MS/MS analysis was performed on the Q-Exactive Plus mass spectrometer (Thermo Scientific) coupled to a Dionex Ultimate 3000 nanoHPLC (Thermo Scientific). Peptides were ionized on a coated PicoTip emitter 10 µm tip size (New Objective, USA) at 1,7 kV spray voltage and 250 °C source temperature. MS Data was acquired from 1 to 200 min with MS full scans between 350 and 1400 m/z (resolution of 70000 at m/z 200). 15 precursors with the highest intensity and charge states at or above +2 to +8 were fragmented using higher energy collisional dissociation (HCD) with normalized collision energy (NCE) of 27 % (isolation width 3 m/z, resolution 17500 at m/z 200). Dynamic exclusion of precursor masses was applied for 30 s. To reduce carry over from different samples, wash steps in-between samples were performed. HPLC separation quality and the mass accuracy of the mass spectrometer were monitored using a cytochrome c digest.

Peptide identification was carried out using Proteome Discoverer 2.2 (Thermo Scientific) using the SequestHT algorithm operating with the reviewed UniProt S. cerevisiae (ATCC 204508 / S288c) protein database (6,729 sequences, 2018 release). For exopeptidase digestions, enzyme specificity was set to unspecific. Allowed missed cleavages were set to 3.

In the case of aminopeptidase digestions, methionine oxidation [+15.99 Da], 180 labeling (18O(0) [+0 Da], 180(1) [+2.00 Da] and 180(2) [+4.00 Da]) on peptide C-termini were set as variable modifications. Carbamidomethylation of cysteine residues [+57.02 Da] was set to static. Peptides that were solely identified by ¹⁸O(0) were considered to be *in-vivo* C-terminal identifications.

Peptides that contained the C-terminal ethanolamine were considered to be *in-vivo* C-terminal identifications. In the case of carboxypeptidase experiments, variable modifications were set for acetylation [+42.01 Da] and dimethylation of N-termini [+28.03 Da], while dimethylation on lysine residues [+28.03 Da] was set to fixed. GO annotation of proteins was performed using the STRING web server (v. 11.0, accessible at https://string-db.org/) (according to Szklarczyk, D., et al., STRING v11: protein-protein association networks with increased coverage, supporting functional discovery in genome-wide experimental datasets. Nucleic Acids Res, 2019. 47(D1): p. D607-D613). Graphical representations of amino acid sequences that were identified in terminal amino acid sections, also termed sequence logos, were generated using Icelogo (according to Colaert, N., et al., Improved visualization of protein consensus sequences by iceLogo. Nat Methods, 2009. 6(11): p. 786-7).

In the analysis, a total of 3964 unique N-terminal peptide sections were identified from the the carboxypeptidase digestion, and a total of 4257 unique proteins were identified from the C-terminal peptide sections generated by the aminopeptidase digestion. The subsequent assigning of terminal peptide sections having the same terminal amino acid sequence resulted in the identification of N-termini of 2190 original proteins and C-termini of 1562 of original proteins. It was found that the in-vivo terminal peptides constituted about 20 to 25% of the number of identified terminal peptides.

Table 1 shows exemplary terminal peptide sections that were assigned to the protein identified by its accession number. The N-terminal modifications that were found are indicated as N-term dimethyl for dimethylation and N-term acetyl for acetylation. For the protein having accession No. P00330, which is Adh1, different proteoforms were identified, namely in vivo a free N-terminus and an acetylated N-terminus, for the protein having accession No. P38711 (Rps27b) and P00650 (Pgk1), solely a free amino terminus and respectively an acetylated N-terminus were identified.

As shown in Fig. 1 c), a comparative analysis using CNBr or trypsin for generating peptides of the same sample, represented by a total protein extract of a yeast lysate as described in the example for exopeptidase digestions, results in peptide size distributions that are typical for bottom-up (350-2000 Da) proteomics approaches using trypsin and middle-down approaches using CNBr (2000-5000 Da). Exopeptidase digestion resulted in peptide lengths from both bottom-up and middle-down approaches simultaneously, increasing the range of the analytical method.

The number of individual terminal peptides that were identified from the carboxypeptidase digestion and could be assigned to an individual protein terminus, i.e. N-terminal peptides, is shown in Fig. 1 d), the number of individual C-terminal peptides from the aminopeptidase digestion is shown in Fig. 1 e). Therein, terminal peptides corresponding to the terminal amino acid sequences of the proteins entered in the database are termed canonical, identified terminal peptides that were not annotated in the database as protein termini are termed non-canonical. In Fig. 1 d), for the N-terminal peptides a non-modified N-terminus is termed "free", and an acetylated N-terminus is termed "acetylated". From the unique terminal peptide sections that were identified, those having the same terminal amino acid sequence of at least 5 amino acids were classified as belonging to one N-terminus, or one protein. The results show that for each protein of the mixture, several terminal peptide sections forming each a series were identified and could be assigned to the N-termini of individual proteins by the process.

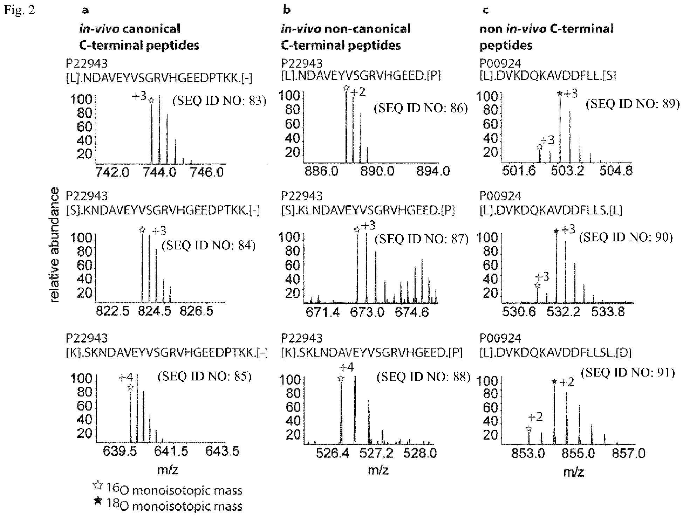

Fig. 2 shows exemplary mass spectrometric results obtained, in a) for the C-terminal peptide sections identified as protein P22943 (Hsp12) as identified in the database (canonical), in b) for the peptide sections identified as protein P22943 but with a C-terminal end that was not represented (non-canonical) in the databank, and in c) for the peptides that were identified as belonging to protein P00924 (Enol) but were identified in their ¹⁸O labelled form (non in-vivo C-terminal peptides). The mass spectrometric data in a) and b) show the ¹⁶O monoisotopic peak (labelled by a white star), which show that no ¹⁸O was introduced from the H₂¹⁸O, and that accordingly the original C-terminal peptide sections were analysed. In c), the varying C-terminal position originated from the AP1 digestion process, but did not represent a C-terminus that was present in the original protein sample, as the isotope pattern is dominated by the ¹⁸O isotope (labelled by a black star). A small proportion of the peptides still contains the ¹⁶O isotope, which creates a distinct pattern that allows easy validation.

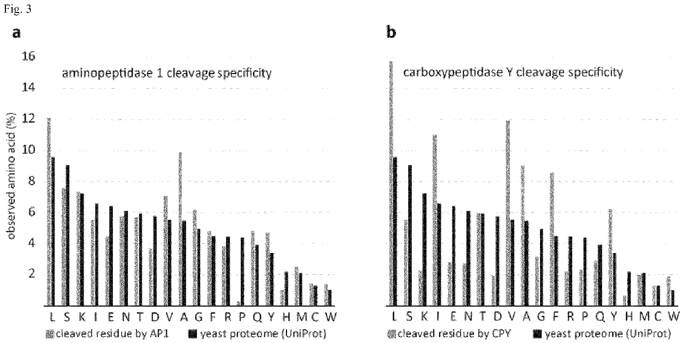

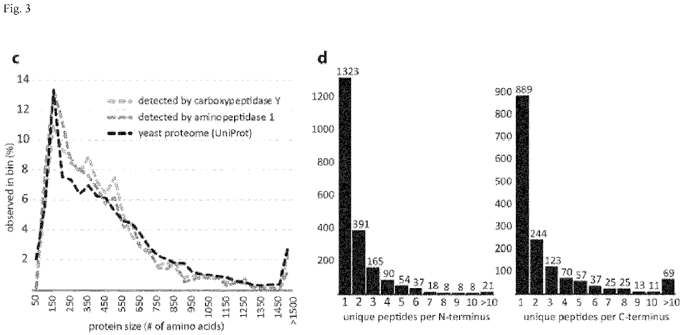

Fig. 3 shows bar charts showing the cleavage preference of a) AP1 and of b) CPY. The occurrence of amino acid residues at the P1 position for AP1 and P1' for CPY is displayed in percent. This is compared to the occurrence of the residue in the S. cerevisiae UniProt database. For AP1, proline is underrepresented, while CPY prefers to hydrolyze hydrophobic amino acids. Fig. 3 c) shows the size distribution of the *S. cerevisiae* proteome as annotated in UniProt. This is compared to the size distributions of proteins detected by terminal peptides using CPY and AP1 digestions indicating no molecular weight bias in the exopeptidase analysis. Fig. 3 d) shows histograms showing the number of unique peptides that characterized a particular protein terminus. N-termini are indicated in the left hand histogram, while C-termini are indicated in the right hand histogram. In total, about 50% of all protein termini were identified with two or more unique peptide sequences.

A comparison of the amino acid sequences determined for the terminal peptide sections of the CPY and AP1 digestion, respectively, with the amino acid sequences of proteins of the databank found that the amino acid sequences determined by the analytical process correspond well with the database sequences, and that acetylated termini were also identified by the analytical process. Further, the analytical process was able to determine terminal amino acid sequences that were not annotated as terminal protein sequences in the utilised database (non-canonical). Bioinformatics analysis of the identified N- and C-termini confirmed that sequence characteristics of canonical protein termini were in-line with the proteins annotated in the yeast database (UniProt).

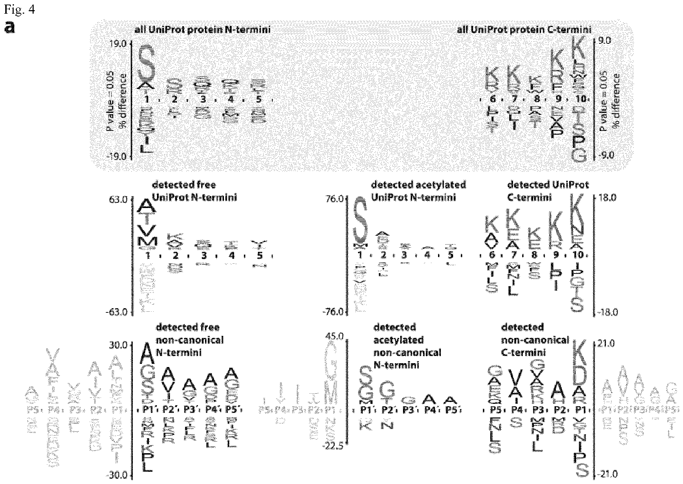

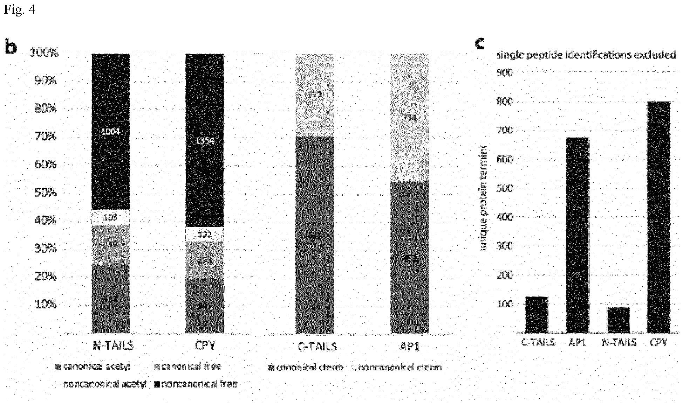

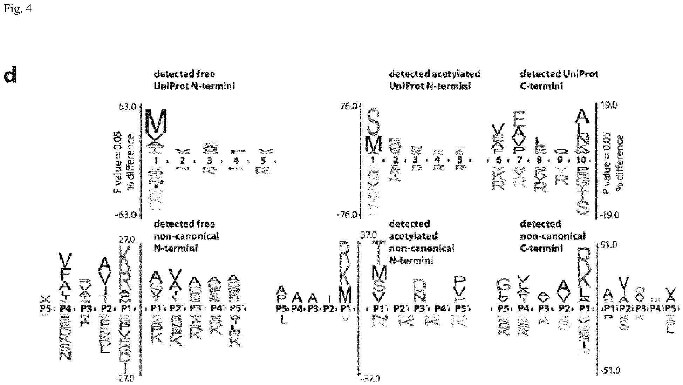

Fig. 4 a) shows sequence logos of identified yeast N- and C-terminal sequences identified using carboxypeptidase and aminopeptidase digestions. Sequence characteristics of identified canonical N- and C-termini match well with their expected sequence characteristics from the UniProt database. In Fig. 4c) the distribution of canonical/non-canonical termini that were identified using exopeptidase digestions is analysed and compared to the established N-/ C-TAILS methodology. Similar distributions were identified but Fig. 3c) shows that the method described by this invention strongly increases confidence in the identified termini by providing multiple unique peptide identification per terminus. This is visualised here by excluding protein termini that were only identified by a single peptide and thus only possess questionable evidence. Fig. 4d) shows sequence logos that were obtained by the N-/C-TAILS methodology. Using such methods, clear biases can be detected that are the result of using sequence-specific endoproteases (trypsin is widely used) to generate terminal protein fragments. As seen for the detected UniProt C-termini (canonical) the prevalence of basic amino acids, which appear in the C-termini annotated in the UniProt database, cannot be reproduced experimentally using C-TAILS. Furthermore, using TAILS the prevalence of lysines (K) and arginines (R) at the P1 position of non-canonical (not annotated in UniProt as a terminus) termini indicates a bias introduced by the use of a sequence specific protease (here trypsin), which is absent in the data generated by the process of the invention using exopeptidases.

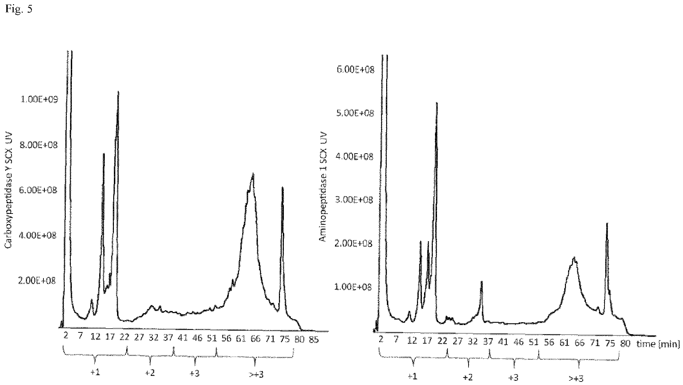

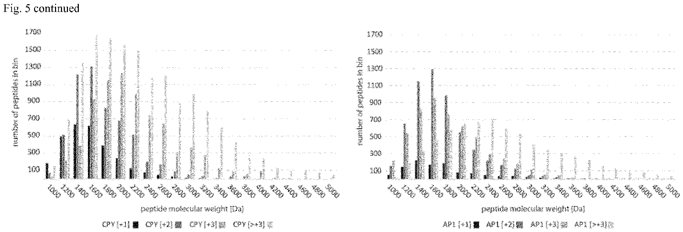

Fig. 5 shows the signal of a UV detector, recorded at 214 nm, for the CPY digestion (left) and for the AP1 digestion (right) at the outlet of the strong ion exchange chromatography column, with fraction numbers and the cation charges indicated. The histograms shown below give the size distribution of peptides that were identified in the different charge fractions. This shows that the molecular weight of peptide sections increases with higher cationic charge, up to 5000 Da, for both digestions. This also shows that size exclusion chromatography of the peptide sections generated by the digestion can be obtained by strong ion exchange chromatography.

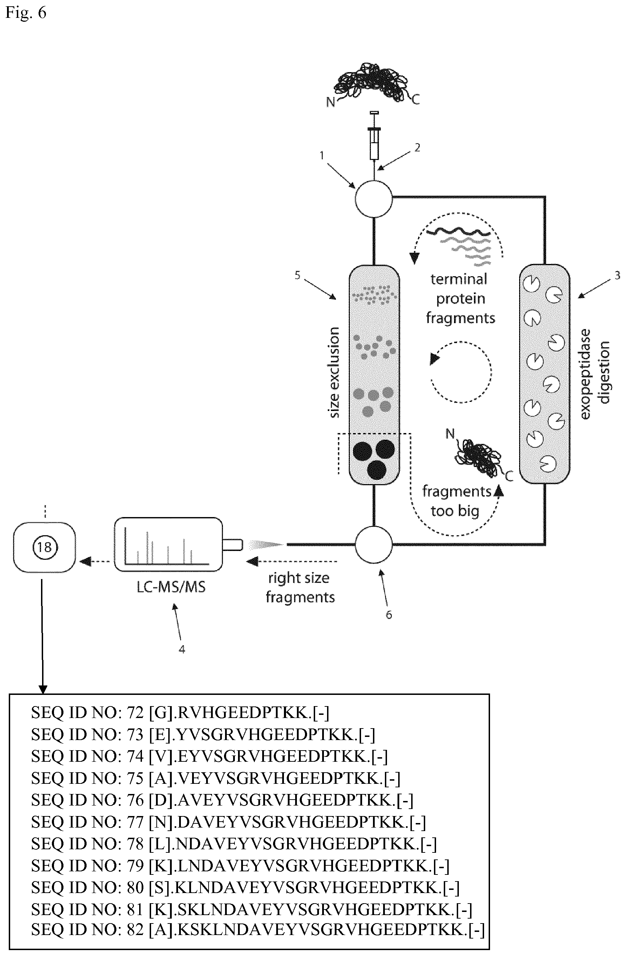

Fig. 6 shows a scheme of an apparatus according to the invention, comprising a size exclusion chromatography column 5 connected downstream of a sample injection port or sample loop 2 connected to a first valve 1. The outlet of the size exclusion chromatography column 5 is connected to a second valve 6, which is controlled to direct peptides of a size suitable for mass spectrometry (right size fragments) to a mass spectrometer 4 (LC-MS/MS), or to direct peptide fragments having a higher molecular size (fragments too big) to a column 3 containing one immobilized exopeptidase (exopeptidase digestion). The outlet of this exopeptidase column 3 is connected to the inlet of the size exclusion chromatography column 5 via the first valve 1. In the mass spectrometer 4 the molecular masses of peptide sections, preferably also fragmentation spectra of peptide sections, are determined. Using comparison with a database 18 that contains pre-determined data, the amino acid sequences of peptide sections are identified, and peptide sections having the same terminal sequence of e.g. a minimum of 5 amino acids can be assigned as belonging to one individual protein terminus (exemplary C-terminal peptide sequences of an individual group are shown).

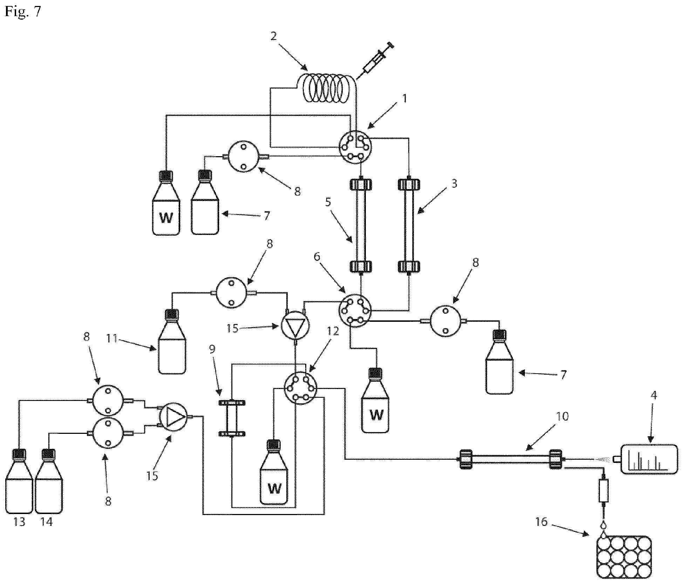

Fig. 7 schematically depicts an apparatus according to the invention, comprising a first valve 1, which is set up for receiving a liquid sample containing a mixture of proteins, e.g. in a sample loop 2 connected to the first valve 1. The inlet of an exopeptidase column 3 which contains one immobilized exopeptidase is connected to the second valve 6, the outlet of the exopeptidase column 3 is connected to valve 1. As generally preferred, a size exclusion chromatography column 5 can be connected to the outlet of the exopeptidase column 3 via a second valve 1. The outlet of the size exclusion chromatography column 5 is connected to the mass spectrometer 4 and, e.g. under the control of a detector, the outlet of the size exclusion chromatography column 5 can be connected to the inlet of the exopeptidase column 3 for returning fractions of peptide sections via valve 6. A container 7 for digestion buffer is connected to the first valve 1 by a pump 8. Between the outlet of the size exclusion chromatography column 5 and the mass spectrometer 4, a third valve 12 connects to a desalting column 9, which is e.g. a C18 desalting column, and to a column 10, which can e.g. be an analytic C18 or cation exchange chromatography column. For provision of digestion buffer, a further container 7 for digestion buffer can be connected to the second valve by an intermediate pump. For providing washing medium e.g. for the desalting column 9, a container 11 for dilute acid can be connected by an intermediate pump 8 and a gradient valve 15 to the third valve 12. For providing ion exchange or reversed -phase medium, containers 13, 14 for medium constituting a medium gradient are connected to the third valve 12 by intermediate pumps 8 and a gradient valve 15. W indicates containers for waste liquids. This describes an "online" version of the apparatus where generated terminal peptides are directly eluted into a mass spectrometer and can be ionized via the electrospray process. By substitution of the mass spectrometer with a fraction collector 16, peptide fractions can be collected "offline" for additional sample handling, which would be necessary if column 10 would be a strong cation exchange column since an additional desalting step is necessary after strong cation exchange chromatography before peptides can be analysed using LC-MS.

The apparatus is set up for receiving a liquid protein sample in the sample loop 2, and the first valve 1 is set up to guide the liquid sample directly into the exopeptidase column 3, or preferably, the first valve 1 is set up to guide the liquid sample into the size exclusion chromatography column 5, and the second valve 6 is controlled, e.g. by a UV detector, to guide the first peptide fraction exiting, which is the large peptide fraction, into the exopeptidase column 3. Further, the second valve 6 is controlled to guide the smaller peptide fraction, e.g. of 500 to 4000 Da, into a desalting column 9. Concurrently, the larger peptide fraction that was guided into the exopeptidase column 3 is digested to smaller terminal peptide sections, which via the first valve 1 are guided into the sample loop 2 and then into the size exclusion chromatography column 5. The peptides that are guided into the desalting column 9 are acidified, e.g. by pumping in of dilute acid from container 11, and pump 8 and a gradient valve 15 connected to the desalting column 9. Acidification of peptides is a prerequisite for efficient binding of the peptides on the desalting column 9. The peptides eluted from the desalting column can be introduced into a strong cation exchange or reversed-phase column 10, to which a eluent gradient generated from the containers 13, 14 via pumps 8 and a gradient valve 15. Generally preferred, the valves are 6-port valves.

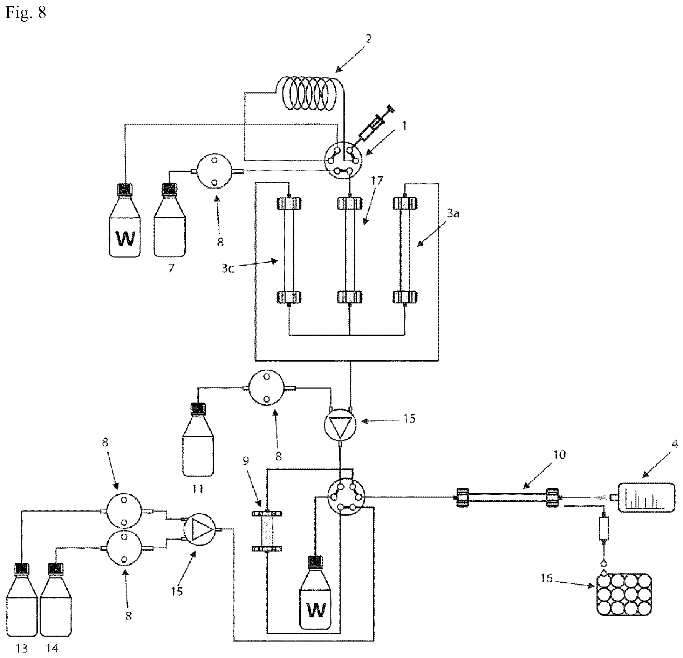

Fig. 8 schematically shows a further embodiment of the apparatus, which connected to the first valve 1 contains two exopeptidase columns 3, one containing an immobilized carboxypeptidase 3c and one containing an immobilized aminopeptidase 3a. Optionally, a column 17 containing immobilized proteinase K can be connected between the first valve 1 and each of the exopeptidase columns 3c, 3a. The digestions from both the 3a and 3c columns are combined and desalted together on desalting column 9. Peptides can then subsequently be eluted via a reversed-phase analytical column or a strong cation exchange column 10 into a fraction collector 16 ("offline analysis"). Alternatively, peptide elution can be directly coupled to a mass spectrometer 4 ("online analysis"). This embodiment of the invention allows to characterize the full length of a sample protein or protein mixture by multiple overlapping peptides in an automated fashion. To achieve this, sample proteins are first digested into smaller fragments using an unspecific endoproteases such as protease K. Those fragments are then diverted simultaneously to an aminopeptidase and a carboxypeptidase column, which digest those fragments sequentially and thus produce N- and C-terminal sequence ladders. Those ladders are then again combined and analysed by LC-MS to characterize the entire protein as well as potential protein modification such as acetylation, phosphorylation, methylation, glycosylation ect. using multiple unique peptides.

### Reference numerals:

- 1: first valve
- 2: sample loop or sample injection port
- 3: exopeptidase column
- 3a: exopeptidase column containing immobilized aminopeptidase
- 3c: exopeptidase column containing immobilized carboxypeptidase
- 4: mass spectrometer
- 5: size exclusion chromatography column
- 6: second valve
- 7: container for digestion buffer
- 8: pump
- 9: desalting column
- 10: reversed-phase or strong cation exchange column
- 11: container for dilute acid
- 12: third valve
- 13, 14: container for ion exchange gradient medium
- 15: gradient valve
- 16: fraction collector
- 17: column containing immobilized proteinase K
- 18: computer having access to a database

## Claims

1. Process for analysis of of terminal sections of proteins, the process comprising or consisting of the steps of
- providing an original isolated protein or a mixture of at least 2 original proteins,
- digesting the mixture of proteins by one exopeptidase, which is a carboxypeptidase or an aminopeptidase, to generate terminal peptide sections of the isolated original protein or of the mixture of original proteins, and free amino acids,
- separating the terminal peptide sections from the free amino acids to generate a fraction comprising or consisting of terminal peptide sections,
- isolating a fraction of terminal peptide sections having a molecular size of 500 Da to 4000 Da,
- determining the masses and determining masses of fragmentation spectra of the terminal peptide sections by mass spectrometry,
- comparing the determined masses to pre-determined masses and comparing the determined masses of the fragmentation spectra to pre-determined masses of fragmentation spectra of known peptides to identify the amino acid sequences of the terminal peptide sections,
- identifying terminal peptide sections which have the same terminal amino acid sequence of at least 5 amino acids and assigning the terminal peptide sections having the same sequence of terminal amino acids to an individual group, and for the individual group classifying the terminal peptide sequences as belonging to one individual terminus of an original protein each, wherein the step of assigning the terminal peptide sequences to an original protein is done by comparing the amino acid sequence of the individual terminal peptide sections to a database containing pre-determined amino acid sequences of proteins.

2. Process according to claim 1, **characterized in that** pre-determined masses of peptides and pre-determined masses of the fragmentation spectra of peptides are contained in and retrieved from the same database.

3. Process according to one of the preceding claims, **characterized by** isolating a fraction of terminal peptide sections which are 2+ or 3+ or higher charge cations by chromatography from the fraction of terminal peptide sections.

4. Process according to one of claims 2 to 3, **characterized in that** chromatography is size exclusion chromatography and/or strong cation exchange chromatography.

5. Process according to one of the preceding claims, **characterized by** subjecting fractions of terminal peptide sections having a molecular mass higher than 4000 Da to an additional step of digestion by the one exopeptidase.

6. Process according to one of the preceding claims, **characterized in that** mass spectrometric detection is by ESI-MS/MS.

7. Process according to one of the preceding claims, **characterized in that** the pre-determined amino acid sequences of proteins contained in the database are pre-determined by computing amino acid sequences that are encoded in pre-determined nucleic acid sequences, which preferably contain the genome of an organism.

8. Process according to one of the preceding claims, **characterized in that** the exopeptidase is immobilized on a carrier arranged in a column, and the original protein or the mixture of original proteins is pumped through this column.

9. Process according to one of the preceding claims, **characterized in that** the exopeptidase is an aminopeptidase and the step of digesting the mixture of proteins is in H₂¹⁸O.

10. Process according to one of the preceding claims, **characterized in that** the exopeptidase is carboxypeptidase and free amino groups of original proteins are chemically modified prior to the step of digesting.

11. Apparatus for use in a process according to one of the preceding claims comprising an ESI-MS/MS mass spectrometer (4), at least one column containing one immobilized exopeptidase (3), which is localized in a column (3) connected to a pump (8) and a first valve (1) that is set up for injection of an isolated protein or of a mixture of at least 2 proteins into the at least one column (3), and a buffer container (7) connected to the pump (8), which buffer container (7) is set up for providing buffer for pumping the mixture through the at least one column (3), wherein the outlet of the column (3) is connected to the mass spectrometer (4), which is coupled to a computer for transfer of data of determined masses, the computer having access to a database (18) containing pre-determined mass data of proteins.

12. Apparatus according to claim 11, **characterized by** comprising at least one column (3a) containing an immobilized exopeptidase, which is an aminopeptidase and at least one column (3c) containing an immobilized exopeptidase which is a carboxypeptidase.

13. Apparatus according to one of claims 11 to 12, comprising a size exclusion chromatography column (5) and/or a strong ion exchange chromatography column (10), which is connected between the outlet of the at least one column (3) containing one immobilized exopeptidase and the mass spectrometer (4).

14. Apparatus according to claim 13, comprising a column (17) containing an immobilized unspecific endoproteases, for example proteinase K, which is connected between the first valve (1) and each of the exopeptidase columns (3, 3a, 3c).

15. Apparatus according to one of claims 13 to 14, wherein the outlet of the size exclusion chromatography column (5) and/or of the strong ion exchange chromatography column (10) is connected via a second valve (6) to the inlet of the at least one column (3) containing an immobilized exopeptidase for guiding a fraction exiting from the outlet of the size exclusion chromatography column (5) and/or of the strong ion exchange chromatography column (10) to the inlet of the at least one column (3) containing an immobilized exopeptidase.


SEQUENCE LISTING

<110> Christian-Albrechts-Universität zu Kiel

<120> Analysis of protein termini

<130> K1035-N-EP

<150> EP 19206374.1
<151> 2019-10-30

<160> 91

<170> BiSSAP 1.3.6

<210> 1
<211> 16
<212> PRT
<213> Artificial Sequence

<220>
<223> peptide determined by MS

<400> 1
Met Val Leu Val Gln Asp Leu Leu His Pro Thr Ala Ala Ser Glu Ala
1               5                   10                  15

<210> 2
<211> 17
<212> PRT
<213> Artificial Sequence

<220>
<223> peptide determined by MS

<400> 2
Met Val Leu Val Gln Asp Leu Leu His Pro Thr Ala Ala Ser Glu Ala
1               5                   10                  15
Arg

<210> 3
<211> 18
<212> PRT
<213> Artificial Sequence

<220>
<223> peptide determined by MS

<400> 3
Met Val Leu Val Gln Asp Leu Leu His Pro Thr Ala Ala Ser Glu Ala
1               5                   10                  15
Arg Lys

<210> 4
<211> 19
<212> PRT

<213> Artificial Sequence

<220>
<223> peptide determined by MS

<400> 4
Met Val Leu Val Gln Asp Leu Leu His Pro Thr Ala Ala Ser Glu Ala
1               5                   10                  15
Arg Lys His

<210> 5
<211> 20
<212> PRT
<213> Artificial Sequence

<220>
<223> peptide determined by MS

<400> 5
Met Val Leu Val Gln Asp Leu Leu His Pro Thr Ala Ala Ser Glu Ala
1               5                   10                  15
Arg Lys His Lys
            20

<210> 6
<211> 22
<212> PRT
<213> Artificial Sequence

<220>
<223> peptide determined by MS

<400> 6
Met Val Leu Val Gln Asp Leu Leu His Pro Thr Ala Ala Ser Glu Ala
1               5                   10                  15
Arg Lys His Lys Leu Lys
            20

<210> 7
<211> 23
<212> PRT
<213> Artificial Sequence

<220>
<223> peptide determined by MS

<400> 7
Met Val Leu Val Gln Asp Leu Leu His Pro Thr Ala Ala Ser Glu Ala
1               5                   10                  15
Arg Lys His Lys Leu Lys Thr
            20

<210> 8
<211> 11
<212> PRT
<213> Artificial Sequence

<220>
<223> peptide determined by MS

<400> 8
Met Ser Leu Ser Ser Lys Leu Ser Val Gln Asp
1               5                   10

<210> 9
<211> 12
<212> PRT
<213> Artificial Sequence


<220>
<223> peptide determined by MS

<400> 9
Met Ser Leu Ser Ser Lys Leu Ser Val Gln Asp Leu
1               5                   10

<210> 10
<211> 17
<212> PRT
<213> Artificial Sequence


<220>
<223> peptide determined by MS

<400> 10
Met Ser Leu Ser Ser Lys Leu Ser Val Gln Asp Leu Asp Leu Lys Asp
1               5                   10                  15
Lys


<210> 11
<211> 18
<212> PRT
<213> Artificial Sequence


<220>
<223> peptide determined by MS

<400> 11
Met Ser Leu Ser Ser Lys Leu Ser Val Gln Asp Leu Asp Leu Lys Asp
1               5                   10                  15
Lys Arg


<210> 12
<211> 19
<212> PRT
<213> Artificial Sequence


<220>
<223> peptide determined by MS

<400> 12
Met Ser Leu Ser Ser Lys Leu Ser Val Gln Asp Leu Asp Leu Lys Asp
1               5                   10                  15
Lys Arg Val

<210> 13
<211> 20
<212> PRT
<213> Artificial Sequence

<220>
<223> peptide determined by MS

<400> 13
Met Ser Leu Ser Ser Lys Leu Ser Val Gln Asp Leu Asp Leu Lys Asp
1               5                   10                  15
Lys Arg Val Phe
            20

<210> 14
<211> 21
<212> PRT
<213> Artificial Sequence

<220>
<223> peptide determined by MS

<400> 14
Met Ser Leu Ser Ser Lys Leu Ser Val Gln Asp Leu Asp Leu Lys Asp
1               5                   10                  15
Lys Arg Val Phe Ile
            20

<210> 15
<211> 19
<212> PRT
<213> Artificial Sequence

<220>
<223> peptide determined by MS

<400> 15
Met Ser Ile Pro Glu Thr Gln Lys Gly Val Ile Phe Tyr Glu Ser His
1               5                   10                  15
Gly Lys Leu

<210> 16
<211> 20
<212> PRT
<213> Artificial Sequence

<220>
<223> peptide determined by MS

<400> 16
Met Ser Ile Pro Glu Thr Gln Lys Gly Val Ile Phe Tyr Glu Ser His
1               5                   10                  15
Gly Lys Leu Glu
            20

<210> 17

<211> 24
<212> PRT
<213> Artificial Sequence

<220>
<223> peptide determined by MS

<400> 17
Met Ser Ile Pro Glu Thr Gln Lys Gly Val Ile Phe Tyr Glu Ser His
1               5                   10                  15
Gly Lys Leu Glu Tyr Lys Asp Ile
            20

<210> 18
<211> 30
<212> PRT
<213> Artificial Sequence

<220>
<223> peptide determined by MS

<400> 18
Met Ser Ile Pro Glu Thr Gln Lys Gly Val Ile Phe Tyr Glu Ser His
1               5                   10                  15
Gly Lys Leu Glu Tyr Lys Asp Ile Pro Val Pro Lys Pro Lys
            20                  25                  30

<210> 19
<211> 31
<212> PRT
<213> Artificial Sequence

<220>
<223> peptide determined by MS

<400> 19
Met Ser Ile Pro Glu Thr Gln Lys Gly Val Ile Phe Tyr Glu Ser His
1               5                   10                  15
Gly Lys Leu Glu Tyr Lys Asp Ile Pro Val Pro Lys Pro Lys Ala
            20                  25                  30

<210> 20
<211> 32
<212> PRT
<213> Artificial Sequence

<220>
<223> peptide determined by MS

<400> 20
Met Ser Ile Pro Glu Thr Gln Lys Gly Val Ile Phe Tyr Glu Ser His
1               5                   10                  15
Gly Lys Leu Glu Tyr Lys Asp Ile Pro Val Pro Lys Pro Lys Ala Asn
            20                  25                  30

<210> 21
<211> 34

<212> PRT
<213> Artificial Sequence

<220>
<223> peptide determined by MS

<400> 21
Met Ser Ile Pro Glu Thr Gln Lys Gly Val Ile Phe Tyr Glu Ser His
1               5                   10                  15
Gly Lys Leu Glu Tyr Lys Asp Ile Pro Val Pro Lys Pro Lys Ala Asn
            20                  25                  30
Glu Leu

<210> 22
<211> 18
<212> PRT
<213> Artificial Sequence

<220>
<223> peptide determined by MS

<400> 22
Met Ser Ile Pro Glu Thr Gln Lys Gly Val Ile Phe Tyr Glu Ser His
1               5                   10                  15
Gly Lys

<210> 23
<211> 19
<212> PRT
<213> Artificial Sequence

<220>
<223> peptide determined by MS

<400> 23
Met Ser Ile Pro Glu Thr Gln Lys Gly Val Ile Phe Tyr Glu Ser His
1               5                   10                  15
Gly Lys Leu

<210> 24
<211> 20
<212> PRT
<213> Artificial Sequence

<220>
<223> peptide determined by MS

<400> 24
Met Ser Ile Pro Glu Thr Gln Lys Gly Val Ile Phe Tyr Glu Ser His
1               5                   10                  15
Gly Lys Leu Glu
            20

<210> 25
<211> 24
<212> PRT

<213> Artificial Sequence

<220>
<223> peptide determined by MS

<400> 25
Met Ser Ile Pro Glu Thr Gln Lys Gly Val Ile Phe Tyr Glu Ser His
1               5                   10                  15
Gly Lys Leu Glu Tyr Lys Asp Ile
            20

<210> 26
<211> 26
<212> PRT
<213> Artificial Sequence

<220>
<223> peptide determined by MS

<400> 26
Met Ser Ile Pro Glu Thr Gln Lys Gly Val Ile Phe Tyr Glu Ser His
1               5                   10                  15
Gly Lys Leu Glu Tyr Lys Asp Ile Pro Val
            20                  25

<210> 27
<211> 30
<212> PRT
<213> Artificial Sequence

<220>
<223> peptide determined by MS

<400> 27
Met Ser Ile Pro Glu Thr Gln Lys Gly Val Ile Phe Tyr Glu Ser His
1               5                   10                  15
Gly Lys Leu Glu Tyr Lys Asp Ile Pro Val Pro Lys Pro Lys
            20                  25                  30

<210> 28
<211> 31
<212> PRT
<213> Artificial Sequence

<220>
<223> peptide determined by MS

<400> 28
Met Ser Ile Pro Glu Thr Gln Lys Gly Val Ile Phe Tyr Glu Ser His
1               5                   10                  15
Gly Lys Leu Glu Tyr Lys Asp Ile Pro Val Pro Lys Pro Lys Ala
            20                  25                  30

<210> 29
<211> 22
<212> PRT
<213> Artificial Sequence

<220>
<223> peptide determined by MS

<400> 29
Thr Val Leu Cys Thr Pro Thr Gly Gly Lys Ala Lys Leu Ser Glu Gly
1               5                   10                  15
Thr Ser Phe Arg Arg Lys
            20

<210> 30
<211> 21
<212> PRT
<213> Artificial Sequence


<220>
<223> peptide determined by MS

<400> 30
Val Leu Cys Thr Pro Thr Gly Gly Lys Ala Lys Leu Ser Glu Gly Thr
1               5                   10                  15
Ser Phe Arg Arg Lys
            20

<210> 31
<211> 20
<212> PRT
<213> Artificial Sequence


<220>
<223> peptide determined by MS

<400> 31
Leu Cys Thr Pro Thr Gly Gly Lys Ala Lys Leu Ser Glu Gly Thr Ser
1               5                   10                  15
Phe Arg Arg Lys
            20

<210> 32
<211> 19
<212> PRT
<213> Artificial Sequence


<220>
<223> peptide determined by MS

<400> 32
Cys Thr Pro Thr Gly Gly Lys Ala Lys Leu Ser Glu Gly Thr Ser Phe
1               5                   10                  15
Arg Arg Lys


<210> 33
<211> 17
<212> PRT
<213> Artificial Sequence


<220>
<223> peptide determined by MS

<400> 33
Pro Thr Gly Gly Lys Ala Lys Leu Ser Glu Gly Thr Ser Phe Arg Arg
1               5                   10                  15
Lys

<210> 34
<211> 16
<212> PRT
<213> Artificial Sequence

<220>
<223> peptide determined by MS

<400> 34
Thr Gly Gly Lys Ala Lys Leu Ser Glu Gly Thr Ser Phe Arg Arg Lys
1               5                   10                  15

<210> 35
<211> 15
<212> PRT
<213> Artificial Sequence

<220>
<223> peptide determined by MS

<400> 35
Gly Gly Lys Ala Lys Leu Ser Glu Gly Thr Ser Phe Arg Arg Lys
1               5                   10                  15

<210> 36
<211> 17
<212> PRT
<213> Artificial Sequence

<220>
<223> peptide determined by MS

<400> 36
Leu Leu Glu Gly Lys Glu Leu Pro Gly Val Ala Phe Leu Ser Glu Lys
1               5                   10                  15
Lys

<210> 37
<211> 16
<212> PRT
<213> Artificial Sequence

<220>
<223> peptide determined by MS

<400> 37
Leu Glu Gly Lys Glu Leu Pro Gly Val Ala Phe Leu Ser Glu Lys Lys
1               5                   10                  15

<210> 38
<211> 15
<212> PRT
<213> Artificial Sequence

<220>
<223> peptide determined by MS

<400> 38
Glu Gly Lys Glu Leu Pro Gly Val Ala Phe Leu Ser Glu Lys Lys
1               5                   10                  15

<210> 39
<211> 14
<212> PRT
<213> Artificial Sequence

<220>
<223> peptide determined by MS

<400> 39
Gly Lys Glu Leu Pro Gly Val Ala Phe Leu Ser Glu Lys Lys
1               5                   10

<210> 40
<211> 13
<212> PRT
<213> Artificial Sequence

<220>
<223> peptide determined by MS

<400> 40
Lys Glu Leu Pro Gly Val Ala Phe Leu Ser Glu Lys Lys
1               5                   10

<210> 41
<211> 12
<212> PRT
<213> Artificial Sequence

<220>
<223> peptide determined by MS

<400> 41
Glu Leu Pro Gly Val Ala Phe Leu Ser Glu Lys Lys
1               5                   10

<210> 42
<211> 11
<212> PRT
<213> Artificial Sequence

<220>
<223> peptide determined by MS

<400> 42

Leu Pro Gly Val Ala Phe Leu Ser Glu Lys Lys
1               5                   10

<210> 43
<211> 26
<212> PRT
<213> Artificial Sequence

<220>
<223> peptide determined by MS

<400> 43
Leu Ser Thr Leu Pro Glu Ile Tyr Glu Lys Met Glu Lys Gly Gln Ile
1               5                   10                  15
Val Gly Arg Tyr Val Val Asp Thr Ser Lys
            20                  25

<210> 44
<211> 25
<212> PRT
<213> Artificial Sequence

<220>
<223> peptide determined by MS

<400> 44
Ser Thr Leu Pro Glu Ile Tyr Glu Lys Met Glu Lys Gly Gln Ile Val
1               5                   10                  15
Gly Arg Tyr Val Val Asp Thr Ser Lys
            20                  25

<210> 45
<211> 24
<212> PRT
<213> Artificial Sequence

<220>
<223> peptide determined by MS

<400> 45
Thr Leu Pro Glu Ile Tyr Glu Lys Met Glu Lys Gly Gln Ile Val Gly
1               5                   10                  15
Arg Tyr Val Val Asp Thr Ser Lys
            20

<210> 46
<211> 19
<212> PRT
<213> Artificial Sequence

<220>
<223> peptide determined by MS

<400> 46
Tyr Glu Lys Met Glu Lys Gly Gln Ile Val Gly Arg Tyr Val Val Asp
1               5                   10                  15
Thr Ser Lys

<210> 47
<211> 16
<212> PRT
<213> Artificial Sequence

<220>
<223> peptide determined by MS

<400> 47
Met Glu Lys Gly Gln Ile Val Gly Arg Tyr Val Val Asp Thr Ser Lys
1               5                   10                  15

<210> 48
<211> 15
<212> PRT
<213> Artificial Sequence

<220>
<223> peptide determined by MS

<400> 48
Glu Lys Gly Gln Ile Val Gly Arg Tyr Val Val Asp Thr Ser Lys
1               5                   10                  15

<210> 49
<211> 14
<212> PRT
<213> Artificial Sequence

<220>
<223> peptide determined by MS

<400> 49
Lys Gly Gln Ile Val Gly Arg Tyr Val Val Asp Thr Ser Lys
1               5                   10

<210> 50
<211> 11
<212> PRT
<213> Artificial Sequence

<220>
<223> peptide determined by MS

<400> 50
Met Val Leu Val Gln Asp Leu Leu His Pro Thr
1               5                   10

<210> 51
<211> 12
<212> PRT
<213> Artificial Sequence

<220>
<223> peptide determined by MS

<400> 51
Met Val Leu Val Gln Asp Leu Leu His Pro Thr Ala
1               5                   10

<210> 52
<211> 16
<212> PRT
<213> Artificial Sequence

<220>
<223> peptide determined by MS

<400> 52
Met Val Leu Val Gln Asp Leu Leu His Pro Thr Ala Ala Ser Glu Ala
1               5                   10                  15

<210> 53
<211> 17
<212> PRT
<213> Artificial Sequence

<220>
<223> peptide determined by MS

<400> 53
Met Val Leu Val Gln Asp Leu Leu His Pro Thr Ala Ala Ser Glu Ala
1               5                   10                  15
Arg

<210> 54
<211> 18
<212> PRT
<213> Artificial Sequence

<220>
<223> peptide determined by MS

<400> 54
Met Val Leu Val Gln Asp Leu Leu His Pro Thr Ala Ala Ser Glu Arg
1               5                   10                  15
Lys His

<210> 55
<211> 19
<212> PRT
<213> Artificial Sequence

<220>
<223> peptide determined by MS

<400> 55
Met Val Leu Val Gln Asp Leu Leu His Pro Thr Ala Ala Ser Glu Arg
1               5                   10                  15
Lys His Lys

<210> 56
<211> 21
<212> PRT
<213> Artificial Sequence

<220>
<223> peptide determined by MS

<400> 56
Met Val Leu Val Gln Asp Leu Leu His Pro Thr Ala Ala Ser Glu Arg
1               5                   10                  15
Lys His Lys Leu Lys
            20

<210> 57
<211> 22
<212> PRT
<213> Artificial Sequence

<220>
<223> peptide determined by MS

<400> 57
Met Val Leu Val Gln Asp Leu Leu His Pro Thr Ala Ala Ser Glu Arg
1               5                   10                  15
Lys His Lys Leu Lys Thr
            20

<210> 58
<211> 23
<212> PRT
<213> Artificial Sequence

<220>
<223> peptide determined by MS

<400> 58
Met Val Leu Val Gln Asp Leu Leu His Pro Thr Ala Ala Ser Glu Arg
1               5                   10                  15
Lys His Lys Leu Lys Thr Leu
            20

<210> 59
<211> 24
<212> PRT
<213> Artificial Sequence

<220>
<223> peptide determined by MS

<400> 59
Met Val Leu Val Gln Asp Leu Leu His Pro Thr Ala Ala Ser Glu Arg
1               5                   10                  15
Lys His Lys Leu Lys Thr Leu Val
            20

<210> 60
<211> 25

<212> PRT
<213> Artificial Sequence

<220>
<223> peptide determined by MS

<400> 60
Met Val Leu Val Gln Asp Leu Leu His Pro Thr Ala Ala Ser Glu Arg
1               5                   10                  15
Lys His Lys Leu Lys Thr Leu Val Gln
            20                  25

<210> 61
<211> 12
<212> PRT
<213> Artificial Sequence

<220>
<223> peptide determined by MS

<400> 61
Gly Arg Val His Gly Glu Glu Asp Pro Thr Lys Lys
1               5                   10

<210> 62
<211> 16
<212> PRT
<213> Artificial Sequence

<220>
<223> peptide determined by MS

<400> 62
Glu Tyr Val Ser Gly Arg Val His Gly Glu Glu Asp Pro Thr Lys Lys
1               5                   10                  15

<210> 63
<211> 17
<212> PRT
<213> Artificial Sequence

<220>
<223> peptide determined by MS

<400> 63
Val Glu Tyr Val Ser Gly Arg Val His Gly Glu Glu Asp Pro Thr Lys
1               5                   10                  15
Lys

<210> 64
<211> 18
<212> PRT
<213> Artificial Sequence

<220>

<223> peptide determined by MS

<400> 64
Ala Val Glu Tyr Val Ser Gly Arg Val His Gly Glu Glu Asp Pro Thr
1               5                   10                  15
Lys Lys


<210> 65
<211> 19
<212> PRT
<213> Artificial Sequence


<220>
<223> peptide determined by MS

<400> 65
Asp Ala Val Glu Tyr Val Ser Gly Arg Val His Gly Glu Glu Asp Pro
1               5                   10                  15
Thr Lys Lys


<210> 66
<211> 20
<212> PRT
<213> Artificial Sequence


<220>
<223> peptide determined by MS

<400> 66
Asn Asp Ala Val Glu Tyr Val Ser Gly Arg Val His Gly Glu Glu Asp
1               5                   10                  15
Pro Thr Lys Lys
            20

<210> 67
<211> 21
<212> PRT
<213> Artificial Sequence


<220>
<223> peptide determined by MS

<400> 67
Leu Asn Asp Ala Val Glu Tyr Val Ser Gly Arg Val His Gly Glu Glu
1               5                   10                  15
Asp Pro Thr Lys Lys
            20

<210> 68
<211> 22
<212> PRT
<213> Artificial Sequence


<220>
<223> peptide determined by MS

<400> 68

Lys Leu Asn Asp Ala Val Glu Tyr Val Ser Gly Arg Val His Gly Glu
1               5                   10                  15
Glu Asp Pro Thr Lys Lys
            20

<210> 69
<211> 23
<212> PRT
<213> Artificial Sequence

<220>
<223> peptide determined by MS

<400> 69
Ser Lys Leu Asn Asp Ala Val Glu Tyr Val Ser Gly Arg Val His Gly
1               5                   10                  15
Glu Glu Asp Pro Thr Lys Lys
            20

<210> 70
<211> 24
<212> PRT
<213> Artificial Sequence

<220>
<223> peptide determined by MS

<400> 70
Lys Ser Lys Leu Asn Asp Ala Val Glu Tyr Val Ser Gly Arg Val His
1               5                   10                  15
Gly Glu Glu Asp Pro Thr Lys Lys
            20

<210> 71
<211> 25
<212> PRT
<213> Artificial Sequence

<220>
<223> peptide determined by MS

<400> 71
Ala Lys Ser Lys Leu Asn Asp Ala Val Glu Tyr Val Ser Gly Arg Val
1               5                   10                  15
His Gly Glu Glu Asp Pro Thr Lys Lys
            20                  25

<210> 72
<211> 12
<212> PRT
<213> Artificial Sequence

<220>
<223> peptide determined by MS

<400> 72
Gly Arg Val His Gly Glu Glu Asp Pro Thr Lys Lys
1               5                   10

<210> 73
<211> 16
<212> PRT
<213> Artificial Sequence

<220>
<223> peptide determined by MS

<400> 73
Glu Tyr Val Ser Gly Arg Val His Gly Glu Glu Asp Pro Thr Lys Lys
1               5                   10                  15

<210> 74
<211> 17
<212> PRT
<213> Artificial Sequence

<220>
<223> peptide determined by MS

<400> 74
Val Glu Tyr Val Ser Gly Arg Val His Gly Glu Glu Asp Pro Thr Lys
1               5                   10                  15
Lys

<210> 75
<211> 18
<212> PRT
<213> Artificial Sequence

<220>
<223> peptide determined by MS

<400> 75
Ala Val Glu Tyr Val Ser Gly Arg Val His Gly Glu Glu Asp Pro Thr
1               5                   10                  15
Lys Lys

<210> 76
<211> 19
<212> PRT
<213> Artificial Sequence

<220>
<223> peptide determined by MS

<400> 76
Asp Ala Val Glu Tyr Val Ser Gly Arg Val His Gly Glu Glu Asp Pro
1               5                   10                  15
Thr Lys Lys

<210> 77
<211> 20
<212> PRT

<213> Artificial Sequence

<220>
<223> peptide determined by MS

<400> 77
Asn Asp Ala Val Glu Tyr Val Ser Gly Arg Val His Gly Glu Glu Asp
1               5                   10                  15
Pro Thr Lys Lys
            20

<210> 78
<211> 21
<212> PRT
<213> Artificial Sequence

<220>
<223> peptide determined by MS

<400> 78
Leu Asn Asp Ala Val Glu Tyr Val Ser Gly Arg Val His Gly Glu Glu
1               5                   10                  15
Asp Pro Thr Lys Lys
            20

<210> 79
<211> 22
<212> PRT
<213> Artificial Sequence

<220>
<223> peptide determined by MS

<400> 79
Lys Leu Asn Asp Ala Val Glu Tyr Val Ser Gly Arg Val His Gly Glu
1               5                   10                  15
Glu Asp Pro Thr Lys Lys
            20

<210> 80
<211> 23
<212> PRT
<213> Artificial Sequence

<220>
<223> peptide determined by MS

<400> 80
Ser Lys Leu Asn Asp Ala Val Glu Tyr Val Ser Gly Arg Val His Gly
1               5                   10                  15
Glu Glu Asp Pro Thr Lys Lys
            20

<210> 81
<211> 24
<212> PRT
<213> Artificial Sequence

<220>
<223> peptide determined by MS

<400> 81
Lys Ser Lys Leu Asn Asp Ala Val Glu Tyr Val Ser Gly Arg Val His
1               5                   10                  15
Gly Glu Glu Asp Pro Thr Lys Lys
            20

<210> 82
<211> 25
<212> PRT
<213> Artificial Sequence


<220>
<223> peptide determined by MS

<400> 82
Ala Lys Ser Lys Leu Asn Asp Ala Val Glu Tyr Val Ser Gly Arg Val
1               5                   10                  15
His Gly Glu Glu Asp Pro Thr Lys Lys
            20                  25

<210> 83
<211> 21
<212> PRT
<213> Artificial Sequence


<220>
<223> peptide determined by MS

<400> 83
Leu Asn Asp Ala Asn Val Tyr Val Ser Gly Arg Val His Gly Glu Glu
1               5                   10                  15
Asp Pro Thr Lys Lys
            20

<210> 84
<211> 22
<212> PRT
<213> Artificial Sequence


<220>
<223> peptide determined by MS

<400> 84
Ser Lys Asn Asp Ala Asn Val Tyr Val Ser Gly Arg Val His Gly Glu
1               5                   10                  15
Glu Asp Pro Thr Lys Lys
            20

<210> 85
<211> 23
<212> PRT
<213> Artificial Sequence


<220>
<223> peptide determined by MS

<400> 85
Lys Ser Lys Asn Asp Ala Asn Val Tyr Val Ser Gly Arg Val His Gly
1               5                   10                  15
Glu Glu Asp Pro Thr Lys Lys
            20

<210> 86
<211> 18
<212> PRT
<213> Artificial Sequence

<220>
<223> peptide determined by MS

<400> 86
Leu Asn Asp Ala Asn Val Tyr Val Ser Gly Arg Val His Gly Glu Glu
1               5                   10                  15
Asp Pro

<210> 87
<211> 19
<212> PRT
<213> Artificial Sequence

<220>
<223> peptide determined by MS

<400> 87
Ser Lys Asn Asp Ala Asn Val Tyr Val Ser Gly Arg Val His Gly Glu
1               5                   10                  15
Glu Asp Pro

<210> 88
<211> 20
<212> PRT
<213> Artificial Sequence

<220>
<223> peptide determined by MS

<400> 88
Lys Ser Lys Asn Asp Ala Asn Val Tyr Val Ser Gly Arg Val His Gly
1               5                   10                  15
Glu Glu Asp Pro
            20

<210> 89
<211> 15
<212> PRT
<213> Artificial Sequence

<220>
<223> peptide determined by MS

<400> 89
Leu Asp Val Lys Asp Gln Lys Ala Val Asp Asp Phe Leu Leu Ser
1               5                   10                  15

<210> 90
<211> 16
<212> PRT
<213> Artificial Sequence

<220>
<223> peptide determined by MS

<400> 90
Leu Asp Val Lys Asp Gln Lys Ala Val Asp Asp Phe Leu Leu Ser Leu
1               5                   10                  15

<210> 91
<211> 17
<212> PRT
<213> Artificial Sequence

<220>
<223> peptide determined by MS

<400> 91
Leu Asp Val Lys Asp Gln Lys Ala Val Asp Asp Phe Leu Leu Ser Leu
1               5                   10                  15
Asp